# EUROPÄISCHE PATENTSCHRIFT

(11) **EP 2 417 940 B1**
(45) Veröffentlichungstag und Bekanntmachung des Hinweises auf die Patenterteilung: **10.06.2015**
(21) Anmeldenummer: 11008151.0
(22) Anmeldetag: 27.01.2009
(51) Int. Cl.: A61F 2/66, A61F 2/68

(54) **Passives orthopädisches Hilfsmittel in Form einer Fussprothese oder Fussorthese**
Passive orthopaedic aid in the form of a foot prosthetic or orthotic
Produit d'aide orthopédique passif sous la forme d'une prothèse de pied ou orthèse de pied

(30) Priorität: 07.02.2008 DE 102008008281
(43) Veröffentlichungstag der Anmeldung: 15.02.2012
(62) Teilanmeldung aus: 09001076.0
(73) Patentinhaber: Otto Bock HealthCare GmbH, 37115 Duderstadt (DE)
(72) Erfinder: Mosler, Lüder, dr., 37115 Duderstadt (DE); Pawlik, Roland, 1130 Wien (AT); Kaltenborn, Sven, 37115 Duderstadt (DE); Zarling, Sven, 37115 Duderstadt (DE); Schörg, Joachim, 2353 Guntramsdorf (AT); Schneider, Greg, Minneapolis MN 55403 (US)
(74) Vertreter: Plöger, Jan Manfred

(56) Entgegenhaltungen:
- WO-A1-2008/103917
- WO-A2-2006/000211
- US-A1- 2002 138 153
- US-A1- 2005 192 677
- US-A1- 2006 235 544

## Beschreibung

Die Erfindung betrifft ein passives orthopädisches. Hilfsmittel in Form einer Fußprothese oder Fußorthese mit
(a) einem ersten Teil,
(b) einem zweiten Teil, wobei das erste Teil drehbar über ein Drehgelenk mit einem zweiten Teil verbunden ist,
(c) einer Sensoranordnung zur Messung von Parametern,
   die Rückschlüsse auf momentane Einsatzerfordernisse des Hilfsmittels erlauben,
(d) einer mit der Steueranordnung verbundenen Steuerung zur Bestirn-mung von Einsatzerfordernissen und Erstellung entsprechender Steuersignale,
(e) einer steuerbaren hydraulischen Dämpferanordnung,
   mit der ein auf die Drehbewegung zwischen dem ersten Teil und dem zweien Teil einwirkender Bewegungswiderstand veränderbar ist, und
(f) einer die Steuersignale der Prozessoranordnung umsetzenden Steuerung zur Steuerung der Dämpferanordnung,
   wobei
(g) die Dämpferanordnung ein doppelt wirkender Hydraulikzylinder mit zwei durch einen Kolben voneinander getrennten Hydraulikkammern ist und
(h) die Hydraulikkammern über zwei, eine Strömung der Hydraulikflüssigkeit nur in entgegengesetzten Richtungen zulassenden Verbindungsleitungen verbunden sind, deren Strömungswiderstände durch die Steuerung separat mit jeweils einem eigenen Stellmittel einstellbar sind.

Derartige orthopädische Hilfsmittel sind insbesondere als Fußprothesen bekannt. Das Drehgelenk ist regelmäßig ein Knöchelgelenk, das ein Anschlussteil für eine Unterschenkelprothese mit einem Fußteil verbindet.

Bei einer aus US 2005/0197717 A1 bekannten Fußprothese weist das Fußteil eine elastische Sohlenfeder auf, die sich über die Länge des Fußteils erstreckt. Die durch die Elastizität der Sohlenfeder bewirkte Dämpfung in der Standphase des Gangzyklus wird dabei ergänzt durch eine aktive Verstellung des Winkels des Fußteils relativ zu dem Anschlussteil zum Unterschenkel. Dadurch lässt sich eine Anpassung an einen geneigten Untergrund oder an eine geänderte Absatzhöhe eines getragenen Schuhs realisieren, wobei für die Änderung der Absatzhöhe eine entsprechende manuelle Eingabe in die Steuerung vorgesehen ist. Der verwendete Aktuator ist vorzugsweise ein doppelt wirkender Motor, insbesondere in Form eines Doppelschraubenmotors. Die Verwendung eines doppelt aktiven Aktuators führt zu einem erheblichen Energiebedarf für die Funktion der Prothese, sodass entweder die Verwendung eines großvolumigen Akkumulators mit einer hohen Speicherkapazität oder ein häufiges Nachladen des Akkumulators vorgesehen werden muss.

Eine passive Fußprothese der eingangs erwähnten Art ist durch US 6,443,993 B1 bzw. US 2002/0138153 A1 bekannt. Zwischen einem Fußteil und einem Ansatzteil für einen Unterschenkel sind anterior und posterior vom Knöchelgelenk jeweils ein Dämpfungszylinder vorgesehen, die in einer Tandemanordnung funktionieren und mit einer gemeinsamen Verbindungsleitung miteinander verbunden sind. Bei Belastung der Ferse wird der posteriore Zylinder zusammengedrückt, sodass Hydraulikflüssigkeit durch die Verbindungsleitung in den anterioren Zylinder gelangt. Die Geschwindigkeit der Strömung der Hydraulikflüssigkeit durch die Verbindungsleitung - und damit die Dämpfung der entsprechenden Kompressionsbewegung an der Ferse - wird dadurch eingestellt, dass als hydraulische Flüssigkeit eine magneto-rheologische Flüssigkeit verwendet wird und mit einer Spule ein entsprechendes magnetisches Feld aufgebaut wird, das die Viskosität der magneto-rheologischen Flüssigkeit verändert. Als Sensoren werden dabei ein Absolut-Neigungssensor und ein Bodenkontaktsensor verwendet. Vorgesehen ist dabei, die Dämpfung in der Standphase des Gangzyklus beispielsweise beim durch den Neigungssensor festgestellten Überschreiten der Lotrechten, zu erhöhen, insbesondere von einem ersten Dämpfungspegel auf einen zweiten Dämpfungspegel umzuschalten. Dadurch ist eine gewisse Anpassung der Prothese an Untergrundschrägen und unterschiedliche Absatzhöhen möglich.

In der teilweise als ältere, jedoch nicht vorveröffentlichte Anmeldung geltenden WO 2008/103917 A1 ist eine Fußprothese beschrieben, bei der eine hydraulische Dämpfung dadurch erreicht wird, dass die Bewegung des Knöchelgelenks eine Bewegung eines Kolbens in einem Zylinder steuert, wodurch Hydraulikflüssigkeit aus einer Zylinderkammer in eine andere Zylinderkammer gedrückt wird. Für beide Bewegungsrichtungen des Gelenks sind separate Verbindungsleitungen vorgesehen, in denen der Strömungswiderstand durch einstellbare Ventile separat einstellbar ist. Zusätzlich zu die Dämpfung bewirkenden Kolben ist ein zweiter Kolben vorgesehen, der im Betriebszustand gegenüber dem ersten Kolben unbeweglich verriegelt ist, jedoch bei geöffnetem Ventil in einem Einstellmodus auf eine aktuelle Absatzhöhe einstellbar ist. Diese Einstellung spiegelt eine Nullpunktstellung wider, die im Betrieb unverändert bleibt. Eine Steuerung ist mit Sensoren verbunden, die Beschleunigungssensoren und Knöchelwinkelsensoren darstellten.

Aus der US 2006/0235544 A1 ist eine Fußprothese der eingangs erwähnten Art bekannt. Dabei befindet sich ein Drehgelenk in einem Fußgehäuse, das Hydraulikkammern ausbildet. Mit dem Drehgelenk verbundene Ansätze ragen in die Kammern, die über Leitungen miteinander verbunden sind. In den Leitungen befinden sich einstellbare Ventile, mit denen der Hydraulikfluss kontrollierbar ist. Zwischen den beiden Kammern können zwei Leitungen ausgebildet sein, die mit entgegengesetzt gerichteten Rückschlagventilen ausgestattet sind, sodass der Hydraulikfluss nur in eine Richtung möglich ist. Dadurch bestimmt eine Leitung mit ihrem einstellbaren Ventil den Strömungswiderstand für die Plantarflexion und die andere Leitung den Strömungswiderstand für die Dorsalflexion des Knöchelgelenks. Die Ventile sind durch einen Mikroprozessor steuerbar, der seine Steuersignale beispielsweise aufgrund des Sensorsignals eines Neigungssensors generiert. Andere Sensoren sind nicht offenbart. Insbesondere kann der Strömungswiderstand besonders hoch eingestellt werden und zu einer Blockierung des Drehgelenks führen, wenn der Neigungssensor eine lotrechte Stellung des Unterschenkelteils ermittelt. Für eine Variation der Ventilstellungen innerhalb des Gangzyklus können vorprogrammierte Steuerungsmuster verwendet werden.

Die sich aus dem Stand der Technik ergebende Problemstellung besteht darin, dass die Steuerung einer passiven Fußprothese oder Fußorthese zwar einzelne Anpassungen ermöglicht, gegenüber dem Verhalten eines natürlichen, gesunden Fußes jedoch noch erhebliche Defizite aufweist.

Der vorliegenden Erfindung liegt daher die Aufgabe zugrunde, eine verbesserte Anpassung an das Verhalten eines natürlichen Fußes durch ein passives orthopädisches Hilfsmittel in Form einer Fußprothese oder Fußorthese zu ermöglichen.

Zur Lösung dieser Aufgabe ist erfindungsgemäß ein passives orthopädisches Hilfsmittel in Form einer Fußprothese oder Fußorthese der eingangs erwähnten Art dadurch gekennzeichnet, dass die Prozessoranordnung zur Bestimmung einer aktuellen Nullpunktstellung aus gemessenen Parametern der wenigstens einen Knöchelmomentsensor und einen Neigungs- oder Absolutwinkelsensor aufweisenden Sensoranordnung ausgelegt ist.

Die erfindungsgemäße Fußprothese oder Fußorthese weist somit einen doppelt wirkenden Hydraulikzylinder auf, dessen sich synchron zueinander verändernde Hydraulikkammern über zwei Verbindungsleitungen miteinander verbunden sind, wobei beide Verbindungsleitungen mit eigenen Stellmittel zur Einstellung ihrer Strömungswiderstände versehen sind. Die Stellmittel können dabei insbesondere steuerbare Ventile sein, deren Durchflussquerschnitt - vorzugsweise stufenlos - veränderbar ist. Diese Anordnung bietet die Möglichkeit, während eines Gangzyklus unterschiedliche Strömungswiderstände für den Fluss der Hydraulikflüssigkeit in der einen und in der anderen Richtung schnell und präzise zu realisieren. Darüber hinaus kann die Verwendung der eigenen Stellmittel dazu ausgenutzt werden, einen stetigen Übergang von der Bewegung des Fußteils in die eine Richtung und in die andere Richtung zu gewährleisten. So ist es beispielsweise für die Steuerung des Knöchelgelenks möglich, einen kontrollierten Übergang von der Plantarflexion in die Dorsalflexion beim Übergang der Gewichtsbelastung von der Ferse auf die Fußspitze zu realisieren, andererseits aber bei der Entlastung der Fußspitze beim Übergang von der Standphase in die Schwungphase des Gangzyklus die ausgebildete Dorsalflexion für eine gewisse Zeit beizubehalten, um das Anheben der Fußspitze am Beginn der Schwungphase bis zum Durchschwingen des Beines über eine Mittelposition hinaus beizubehalten, wie dies mit einem natürlichen gesunden Fuß erfolgt, um ein Anstoßen der Zehenspitzen auf dem Boden beim Durchschwingen zu vermeiden.

Erfindungsgemäß sollen die Sensoranordnung und die Prozessoranordnung so ausgelegt sein, dass jeweils eine aktuelle Nullpunktstellung bestimmbar ist. Eine hierfür geeignete Prozessoranordnung besteht beispielsweise aus einem Knöchelmomentsensor und einem Neigungs- bzw. Absolutwinkelsensor, wenn das Drehgelenk die Funktion eines Knöchelgelenks ausübt. Ergänzend hierzu kann vorzugsweise ein Knöchelwinkelsensor vorgesehen sein. Die Bestimmung eines aktuellen Anschlags nach dorsal ist dabei durch die Ermittlung des Absolutwinkels beim Nulldurchgang des Knöchelmoments in der Standphase des Gangzyklus möglich. Dadurch kann die aktuelle Untergrundneigung bzw. die jeweilige Absatzhöhe bei jedem Gangzyklus ohne Zeitverzögerung berücksichtigt werden. Da dabei der Einfluss der Absatzhöhe und der Untergrundneigung in gleicher Weise in das gemessene Signal eingehen, ist es zweckmäßig, den konstanten Einfluss der Absatzhöhe durch eine entsprechende Auswertung der Signale in der Standphase des Gangzyklus oder beim Stehen zu ermitteln. Alternativ hierzu kann die Absatzhöhe auch manuell in die Auswertungseinrichtung eingebbar sein.

Bei einer bevorzugten Ausführungsform der Erfindung können die Strömungswiderstände in den beiden Verbindungsleitungen so groß eingestellt werden, dass das Drehgelenk in einer Stellung arretiert ist. Für die Arretierung des Drehgelenks können daher die Stellmittel verwendet werden, sodass keine gesonderte Arretierungseinrichtung erforderlich ist, beispielsweise um beim Stehen des Trägers des orthopädischen Hilfsmittels eine stabile Unterstützung durch die Prothese oder Orthese zu gewährleisten. Die arretierte Stellung kann dabei der ermittelten Nullpunktstellung entsprechen.

In einer besonders bevorzugten Ausführungsform der Erfindung sind die Teile des Hilfsmittels überwiegend starr ausgebildet. Die Steuerung wenigstens eines überwiegenden Teils des Bewegungsablaufes innerhalb eines Bewegungszyklus erfolgt ohne Einfluss von Materialelastizitäten durch die Steuerung der Strömungswiderstände. Allenfalls kann es zweckmäßig sein, eine gewisse Materialelastizität beim Aufsetzen der Ferse auf den Untergrund beim Gehen auszunutzen.

Mit dem erfindungsgemäßen orthopädischen Hilfsmittel wird somit eine Nullpunktstellung der Drehbewegung bestimmt, von der aus bei einem zyklischen Bewegungsablauf die Bewegungswiderstände für die erste und die zweite Bewegungsrichtung des Drehgelenks, insbesondere des Knöchelgelenks, bestimmt werden. Für ein definiertes Einsatzerfordernis, beispielsweise beim Stehen, wird durch die Einstellung hoher Bewegungswiderstände in beiden Bewegungsrichtungen das Drehgelenk arretiert, vorzugsweise in dem Nullpunkt. Der Bewegungszyklus ist dabei vorzugsweise ein Gangzyklus.

In einer weiter bevorzugten Ausführungsform der Erfindung ist das am Drehgelenk angeschlossene Teil ein Fußteil, das in ein Hauptfußteil und ein Vorderfußteil unterteilt ist, wobei das Vorderfußteil mit dem Hauptfußteil über ein Gelenk verbunden ist. Der Hydraulikzylinder kann dabei an dem Vorderfußteil angelenkt sein, um so das Hauptfußteil in seiner Winkelstellung indirekt, nämlich über das Vorderfußteil, einzustellen.

Erfindungsgemäß ist ein passives orthopädisches Hilfsmittel in Form einer Fußprothese oder Fußorthese mit einem ersten Teil, das drehbar über ein Drehgelenk mit einem zweiten Teil verbunden ist, mit einer Sensoranordnung zur Messung von Parametern, die Rückschlüsse auf momentane Einsatzerfordernisse des Hilfsmittels erlauben, mit einer mit der Steueranordnung verbundenen Steuerung zur Bestimmung von Einsatzerfordernissen und Erstellung entsprechender Steuersignale, mit einer steuerbaren hydraulischen Dämpferanordnung, mit der ein auf die Drehbewegung zwischen dem ersten Teil und dem zweiten Teil einwirkender Bewegungswiderstand veränderbar ist und mit einer die Steuersignale der Prozessoranordnung umsetzenden Steuerung zur Steuerung der Dämpferanordnung, dadurch gekennzeichnet, dass die Dämpferanordnung ein doppelt wirkender Hydraulikzylinder mit zwei durch einen Kolben voneinander getrennten Hydraulikkammern ist und dass die Hydraulikkammern über zwei, eine Strömung der Hydraulikflüssigkeit nur in entgegengesetzten Richtungen zulassenden Verbindungsleitungen verbunden sind, deren Strömungswiderstände durch die Steuerung separat mit jeweils einem eigenen Stellmittel einstellbar sind, dass die Prozessoranordnung zur Bestimmung einer aktuellen Nullpunktstellung aus den gemessenen Parametern der Sensoranordnung ausgelegt ist und dass die Erstellung der Steuersignale für die Strömungswiderstände in den beiden Verbindungsleitungen in Bezug auf die Nullpunktstellung erfolgt.

Erfindungsgemäß ist zudem ein solches orthopädietechnisches Hilfsmittel, bei dem der Strömungswiderstand in zumindest einer Verbindungsleitung so groß einstellbar ist, dass das Drehgelenk in einer beliebigen Stellung arretierbar ist.

Erfindungsgemäß ist zudem ein orthopädietechnisches Hilfsmittel, bei dem die arretierte Stellung der Nullpunktstellung entspricht.

Erfindungsgemäß ist zudem ein orthopädietechnisches Hilfsmittel, bei dem die Nullpunktstellung in Abhängigkeit einer Neigung des Untergrunds bestimmbar ist.

Erfindungsgemäß ist zudem ein orthopädietechnisches Hilfsmittel, bei dem die Nullpunktstellung in Abhängigkeit von einer aktuellen Absatzhöhe eines mit dem Hilfsmittel verwendeten Schuhs bestimmbar ist.

Erfindungsgemäß ist zudem ein orthopädietechnisches Hilfsmittel, bei dem die Teile des Hilfsmittels überwiegend starr ausgebildet sind und dass die Steuerung wenigstens eines überwiegenden Teils des Bewegungsablaufs innerhalb eines Bewegungszyklusses ohne Einfluss von Materialelastizitäten durch die Steuerung der Strömungswiderstände erfolgt.

Erfindungsgemäß ist zudem ein orthopädietechnisches Hilfsmittel, bei dem das Drehgelenk ein Knöchelgelenk ist.

Erfindungsgemäß ist ein Verfahren zur Steuerung eines erfindungsgemäßen orthopädietechnischen Hilfsmittels, bei dem eine Nullpunktstellung der Drehbewegung bestimmt wird, von der aus bei einem zyklischen Bewegungsablauf der Bewegungswiderstand für mindestens eine Bewegungsrichtung bestimmt wird.

Erfindungsgemäß ist zudem ein Verfahren, bei dem das Hilfsmittel für ein definiertes Einsatzerfordernis durch Einstellung hoher Bewegungswiderstände in beiden Bewegungsrichtungen in dem Nullpunkt arretiert wird.

Erfindungsgemäß ist zudem ein Verfahren, das auf ein orthopädietechnisches Hilfsmittel für eine untere Extremität, bei der ein Gangzyklus als steuerbarer Bewegungszyklus benutzt wird, angewendet wird.

Die Erfindung soll im Folgenden anhand eines in der Zeichnung dargestellten Ausführungsbeispiels näher erläutert werden. Es zeigen:
- Figur 1: eine schematische Darstellung eines ersten Ausführungsbeispiels einer erfindungsgemäßen Fußprothese;
- Figur 2: eine schematische Darstellung eines zweiten Ausführungsbeispiels einer erfindungsgemäßen Fußprothese;
- Figur 3: eine schematische Darstellung eines dritten Ausführungsbeispiels einer erfindungsgemäßen Fußprothese;
- Figur 4: eine Draufsicht auf eine konstruktiv detaillierte weitere Ausführungsform einer erfindungsgemäßen Fußprothese;
- Figur 5: einen Vertikalschnitt parallel zur Sagittalebene durch die Fußprothese gemäß Figur 4.

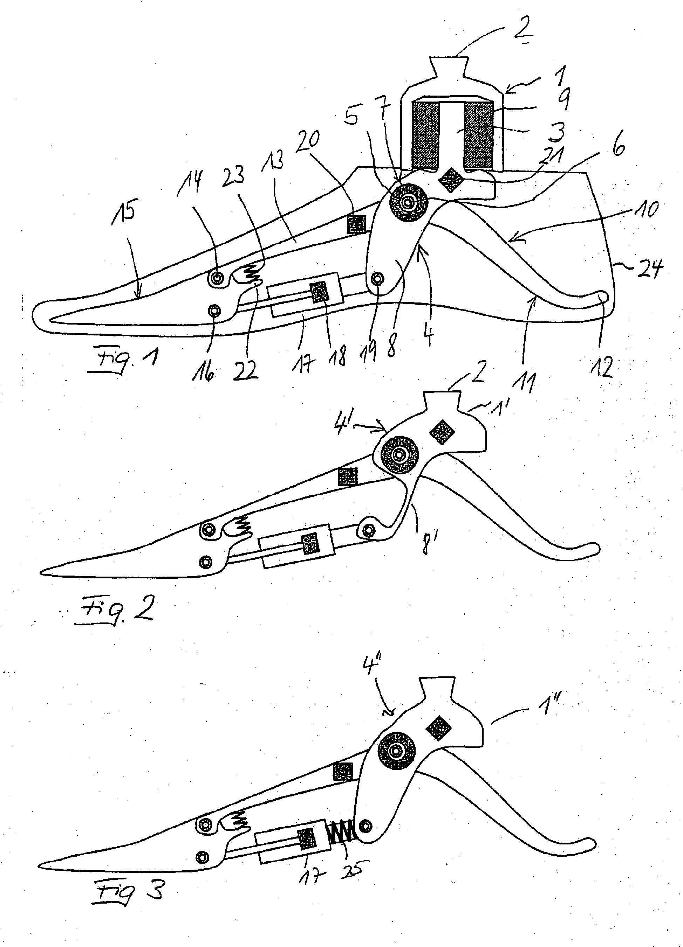

Bei dem in Figur 1 dargestellten Ausführungsbeispiel ist ein Befestigungsstück 1 mit einem Justieransatz 2 in Form eines umgekehrten Pyramidenstumpfes mit vier Schrägflächen ausgebildet. Das Befestigungsstück 1 bildet einen nach unten offenen Topf, in den ein nach oben ragender Steg 3 eines zweiarmigen Hebels 4 hineinragt. Der zweiarmige Hebel ist um ein Drehgelenk 5 drehbar, dessen Drehachse 6 zugleich die Achse eines Knöchelgelenks des künstlichen Fußes bildet. Das Drehgelenk 5 ist mit einem Winkelsensor 7 versehen. Der zweiarmige Hebel 4 weist einen starren, nach unten ragenden Ansatz 8 auf.

In dem nach unten offenen Topf des Befestigungsstücks 1 ist der mit dem Steg 3 gebildete Zwischenraum durch ein relativ steifes, elastisches Material 9 ausgefüllt, sodass die Bewegung des Befestigungsstücks 1 nur etwas bedämpft auf Bewegung des Stegs 3 des zweiarmigen Hebels 4 übertragen wird. Demgemäß vollzieht der Ansatz 8 die Bewegung des Befestigungsstücks 1 nach, jedoch aufgrund des elastischen Materials 9 etwas bedämpft.

Das das Knöchelgelenk bildende Drehgelenk 5 trägt ferner ein Hauptfußteil 10, das sich ebenfalls ein zweiarmiger Hebel mit einem hinteren Hebelarm 11 in den Fersenbereich des Fußes hinein erstreckt, wo der schräg nach hinten und unten verlaufende hintere Hebelarm 11 mit einem etwa horizontal abgerundeten Ende 12 versehen ist.

Das Hauptfußteil weist einen vom Knöchelgelenk 5 nach vorn erstreckten vorderen Hebelarm 13 auf, der sich nahezu geradlinig vom Knöchelgelenk 5 nach vorn etwas schräg nach unten verlaufend erstreckt, sodass das Hauptfußteil 10 zum Knöchelgelenk 5 hin nach oben gewölbt ausgebildet ist und vom Knöchelgelenk 5 nach hinten in den Fersenbereich sowie nach vorne in einen Vorderfußbereich hinein schräg abfällt, wobei der schräge Abfall in den Fersenbereich hinein steiler ist als in den Vorderfußbereich.

Der vordere Hebelarm 13 des Hauptfußteils 10 endet am Beginn des Vorderfußbereichs und trägt dort ein Drehgelenk 14, mit dem ein einen Zehenbereich nachbildendes Vorderfußteil 15 drehbar an dem vorderen Hebelarm 13 des Hauptfußteils angelenkt ist. Das Drehgelenk 14 weist eine Drehachse auf, die parallel zur Drehachse 6 des Knöchelgelenks 5 in horizontaler Richtung verläuft. Da das Vorderfußteil 15 den Zehenbereich eines natürlichen Fußes imitiert, ist es nach vorne hin dreieckförmig mit einer Spitze auslaufend ausgebildet. Unterhalb des Drehgelenks 14 befindet sich am Vorderfußteil 15 ein weiteres Drehgelenk 16, mit dem eine Kolbenstange eines Kolbens 18 eines Hydraulikzylinders 17 an dem Vorderfußteil 15 angelenkt ist. Der Hydraulikzylinder 17 ist mit einem Drehgelenk 19 drehbar an dem freien Ende des nach unten ragenden Ansatzes 8 des zweiarmigen Hebels 4 angelenkt, sodass sich das Drehgelenk 19 unterhalb und etwas nach vorn (in Richtung Vorderfußbereich 15) versetzt zum Knöchelgelenk 5 angeordnet ist.

Das Knöchelgelenk 5 enthält den Winkelsensor 7 für die Messung des Knöchelwinkels, also des Winkels zwischen dem (fluchtend mit dem Unterschenkel ausgerichteten) Steg 3 und dem vorderen Hebelarm 13 des Hauptfußteils 10.

Der vordere Hebelarm 13 des Hauptfußteils 10 trägt ferner einen Neigungssensor 20, der die Neigung relativ zur Erdanziehungskraft (relativ zur Lotrechten) ermittelt. Derartige Neigungssensoren 20, die einen absoluten Neigungswinkel relativ zur Erdbeschleunigung bestimmen, sind als Beschleunigungssensoranordnungen mit oder ohne Gyroskop bekannt.

Der zweiarmige Hebel 4 enthält fluchtend mit dem Justieransatz 2, d.h. fluchtend mit dem (künstlichen) Unterschenkel des Patienten einen Knöchelmomentsensor 21, der das an dieser Stelle wirkende Drehmoment misst.

Das Vorderfußteil 15 ist an seinem hinteren Ende mit einem Lageransatz 22 versehen, der zum Halten einer auf Zug und Druck belastbaren Feder 23 dient, die sich mit ihrem anderen Ende am vorderen Hebelarm 13 des Hauptfußteils 10 abstützt. Die Feder 23 bewirkt eine Rückstellung des Vorderfußteils 15 nach einer Dorsalflexion, wobei die Rückstellgeschwindigkeit durch den Hydraulikzylinder 17 bestimmt wird.

Der Hydraulikzylinder 17 ist als passiver Aktuator ausgebildet, in dem der vom Kolben 18 bewirkte Hydraulikfluss durch (nicht dargestellte) Ventile gesteuert wird, wobei die Ventile selbst nicht nur ein- und ausschaltbar sind, sondern auch für eine definierte Durchflussmenge gesteuert werden können.

Die in Figur 2 dargestellte Ausführungsform entspricht im Wesentlichen der Ausführungsform gemäß Figur 1. Ein Unterschied besteht darin, dass das Befestigungsstück 1' mit dem Justieransatz 2 einstückig ausgebildet ist, sodass eine durch das elastische Material 9 gebildete Elastizität nicht mehr vorhanden ist. Stattdessen ist der nach unten ragende Ansatz 8' des zweiarmigen Hebels 4' mit einer Materialverdünnung ausgebildet, sodass das das Drehgelenk 19 tragende freie Ende des Ansatzes elastisch federnd zum restlichen Material des zweiarmigen Hebels 4' angeordnet ist.

Selbstverständlich weist der künstliche Fuß gemäß der zweiten Ausführungsform ebenso einen kosmetischen Überzug 24 auf, wie die erste Ausführungsform. Dieser kosmetische Überzug 24 ist für die zweite und dritte Ausführungsform jedoch nicht wiederholt dargestellt.

Bei dem künstlichen Fuß gemäß der in Figur 3 dargestellten dritten Ausführungsform ist der zweiarmige Hebel 4" ebenfalls einstückig mit dem Justieransatz 2 ausgebildet. Auch der nach unten ragende Ansatz 8 des zweiarmigen Hebels 4" ist starr wie bei der ersten Ausführungsform. Stattdessen ist der Hydraulikzylinder 17 über eine Spiralfeder 25 elastisch mit dem nach unten ragenden Ansatz 8 des zweiarmigen Hebels 4" verbunden. Dadurch wird in Serie mit der Wirkung des Hydraulikzylinders 17 eine Elastizität verwirklicht, die bei der in Figur 1 dargestellten Ausführungsform durch das elastische Material 9 und bei der in Figur 2 dargestellten Ausführungsform durch den federnden Ansatz 8' realisiert ist. Alle übrigen Teile der dritten Ausführungsform entsprechen denen der ersten Ausführungsform.

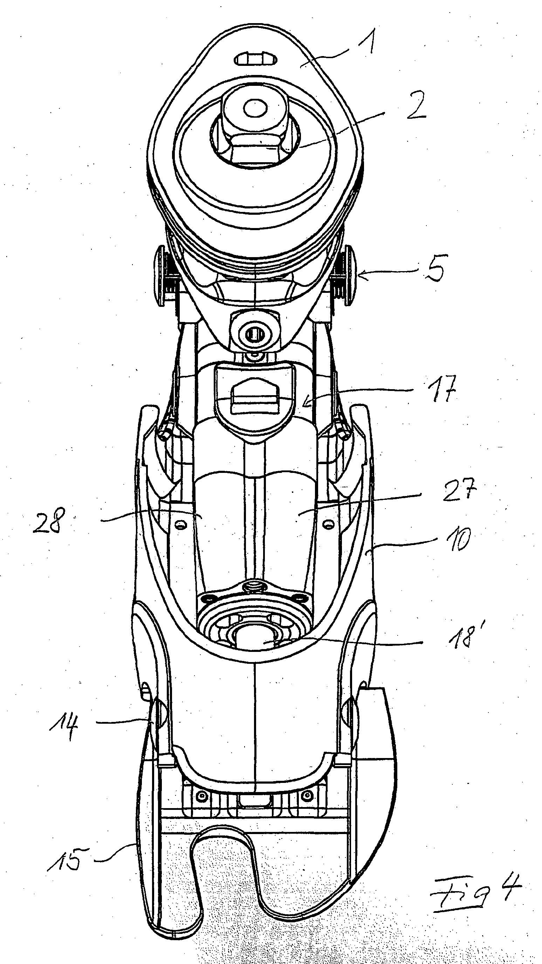

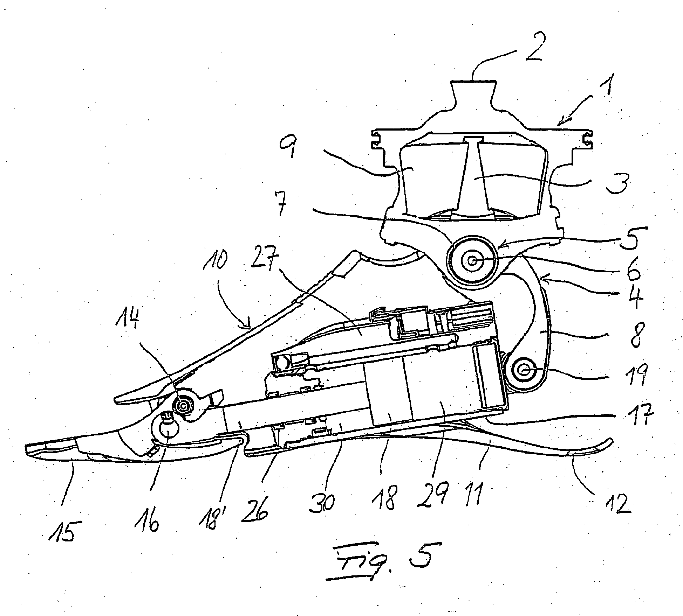

Das in den Figuren 4 und 5 dargestellte Ausführungsbeispiel lässt das Befestigungsstück 1 mit dem pyramidenförmigen Justieransatz 2 erkennen. In dem Befestigungsstück 1 befindet sich das elastische Material 9, das mit dem nach oben ragenden Steg 3 des zweiarmigen Hebels 4 dämpfend zusammenwirkt. Der nach unten ragende Ansatz 8 des zweiarmigen Hebels 4 verläuft in diesem Ausführungsbeispiel in Gehrichtung hinter dem Knöchelgelenk 5 und ist dort über das Drehgelenk 19 an dem Hydraulikzylinder 17 angelenkt. In dem Hydraulikzylinder 17 bewegt sich längsverschiebbar der Kolben 18, der über ein im Hydraulikzylinder 17 befindliches Lager 26 herausgeführt und mit dem weiteren Drehgelenk 16 des Vorderfußteils 15 verbunden ist. Das Knöchelgelenk 5 lagert ferner das Hauptfußteil 10, das hier in Form eines starren Gehäuses ausgebildet ist und einen nach hinten gerichteten elastischen Hebel 11 als Fersenhebel aufweist. Das Fußteil 10 ist mit dem Fersenhebel 11 somit gemeinsam um das Knöchelgelenk 5 relativ zu dem Befestigungsstück 1 und zu dem zweiarmigen Hebel 4 schwenkbar. Die Schwenkbewegung zwischen dem Befestigungsstück 1 und dem Fußteil 10 wird über den zweiarmigen Hebel 4 und den Hydraulikzylinder 17 gesteuert und bedämpft. Die Anlenkung der Kolbenstange 18' des Kolbens 18 an dem Vorderfußteil 15 bewirkt dabei lediglich eine zusätzliche Steuerung des die Zehenplatte bildenden Vorderfußteils 15, die jedoch die Steuerung des Hauptfußteils 10 nur geringfügig modifiziert, da das weitere Drehgelenk 16 in unmittelbarer Nähe des Drehgelenks 14 zwischen dem Vorderfußteil 15 und dem Hauptfußteil 10 angeordnet ist. Der Hydraulikzylinder 17 ist mit zwei Steuerventilen 27, 28 versehen, die auf der Oberseite des Hydraulikzylinders 17 angeordnet sind. Die Steuerventile 27, 28 sind mit Kammern 29, 30 des Hydraulikzylinders 17 beiderseits des Kolbens 18 verbunden, wobei (nicht dargestellte) Rückschlagventile dafür sorgen, dass durch das erste Steuerventil 27 lediglich ein Fluss der Hydraulikflüssigkeit von der unteren Kammer 29 zur vorderen Kammer 30 erfolgen kann, wodurch die Einschubbewegung des Kolbens 18 in den Hydraulikzylinder 17 ermöglicht wird, was einer Plantarflexion des Hauptfußteils 10 gegenüber dem Befestigungsstück 1 entspricht. Der andere Steuerzylinder 28 ermöglicht mit Rückschlagventilen lediglich den Hydraulikfluss von der vorderen Kammer 30 in die hintere Kammer 29, wodurch ein Herausziehen des Kolbens 18 aus dem Hydraulikzylinder 17 ermöglicht wird, also eine Verlängerung des Abstandes zwischen den Drehgelenken 19, 16 stattfindet. Dies entspricht einer Dorsalflexion zwischen dem Befestigungsstück 1 und dem Hauptfußteil 10. Gleichzeitig wird durch die Verschiebung des Drehgelenks 16 gegenüber dem Drehgelenk 14 nach vorn ein Anheben des Vorderfußteils 15 bewirkt.

Die Funktion des künstlichen Fußes ist für die dargestellten Ausführungsformen gleich. Mit der Sensoranordnung zur Messung des Knöchelwinkels, des

Knöchelmoments und des absoluten Neigungswinkels lassen sich die relevanten Funktionszustände des künstlichen Fußes ermitteln und differenzieren, wobei das Signal des Knöchelwinkelsensors zur Ermittlung des Knöchelwinkels (zwischen Befestigungsstück 1, 1', 1" und Hauptfußteil 10) einerseits und die jeweilige Knöchelwinkelgeschwindigkeit andererseits ausgewertet werden.

So kann beispielsweise die Erkennung, ob mit dem künstlichen Fuß gegangen wird oder gestanden wird, dadurch erfolgen, dass die Knöchelwinkelgeschwindigkeit im Nulldurchgang des Knöchelmoments bestimmt wird. Liegt die Knöchelwinkelgeschwindigkeit im Nulldurchgang des Knöchelmoments unter einem Schwellwert, wird dies als "Stehen" erkannt und der Aktuator in Form des Hydraulikzylinders 17 mittels der Ventile auf einen hohen Widerstand eingestellt, durch den ein Dorsalanschlag gebildet werden kann.

Die Erkennung einer abschüssigen Neigung oder der Absatzhöhe wird durch den Neigungssensor 20 im Mittelfußbereich des Hauptfußteils 10 beim Nulldurchgang des Knöchelmoments bestimmt.

Wird das Gehen in der Ebene detektiert, wird das Ventil, das für die Plantarflexion des Fußes zuständig ist, in einer halboffenen Stellung belassen, während das Ventil, das die Dorsalfelxion bestimmt, mit zunehmenden Knöchelwinkel geschlossen wird, um einen Dorsalanschlag zu bilden.

Wird ein Gehen bergauf detektiert, wird dabei eine erweiterte Dorsalflexion des Vorderfußteils 15 zugelassen.

Wird beim Gehen der Fersenauftritt nach der Schwungphase und zu Beginn der Standphase insbesondere durch ein negatives Knöchelmoment detektiert, wird das Ventil für die Plantarflexion so gesteuert, dass es mit zunehmenden Knöchelwinkel in Richtung der Plantarflexion schließt und somit einen Anschlag für die Plantarflexion bildet.

Wird ein Zehenabstoß am Ende der Standphase detektiert (abfallendes Knöchelmoment bei vergrößertem Knöchelwinkel), wird das Ventil für die Dorsalflexion nach einer Totzeit vollständig geöffnet, um durch ein elastisches Element die Anhebung des Vorderfußteils (Zehenanhebung) in der Schwungphase auszulösen.

An diesen Beispielen ist erkennbar, dass die wichtigen Steuerungen eines künstlichen Fußes beim Stehen und beim Gehen auch in Abhängigkeit von der Bodenneigung oder der Absatzhöhe zutreffend vorgenommen werden können, wobei die Steuerung des Bewegungswiderstandes über den Hydraulikzylinder bereits ausreicht.

In einem Ausführungsbeispiel für die Erkennung der Bewegungszustände für die Fußprothese und die daraus resultierende Steuerung werden folgende Funktionen realisiert:

### Unterscheidung Stehen-Gehen

Die Unterscheidung zwischen Stehen und Gehen erfolgt mit folgenden Kriterien:
1. Erkennung einer Schwungphase
   Eine Schwungphase wird dadurch erkannt, dass das Knöchelmoment nahe Null ist, da der Fuß in der Schwungphase unbelastet ist.
   Der Absolutwinkel des Fußteils 10 überschreitet einen Schwellenwert, der für das Stehen individuell definiert sein kann. Ferner überschreitet die Absolutwinkelgeschwindigkeit einen definierten Schwellenwert.
2. Erkennen eines Fersenauftritts im vorgeschwungenen Zustand
   Es wird ein negatives Knöchelmoment (plantarflektierend) detektiert. Das Absolutwinkelsignal entspricht einem vorgeschwungenen Fuß im Vergleich zu einem für das Stehen individuell definierten Schwellenwert.
   Optional kann eine Plantarflexion beim Fersenauftritt über die Knöchelwinkelgeschwindigkeit angezeigt werden.
3. Rückkehr zum Stehen
   Nach einem detektierten Fersenauftritt verbleibt der Absolutwinkel des Fußteils 10 innerhalb eines für das Stehen individuell definierten Schwellenwertes. Alternativ oder ergänzend kann eine aktive Umkehr der Bewegungsrichtung in der mittleren Standphase von dorsal nach plantar als Kriterium für das Stehen erkannt werden.
   Beim detektierten Stehen werden die Steuerventile 27, 28 so eingestellt, dass sich für das Stehen Anschläge nach ventral und dorsal für einen engen Winkel (Nullpunktlage) ergeben. Für den Gangzyklus wird der Anschlag nach dorsal verschoben und die Dämpfungseigenschaften für die Plantar- und Dorsalflexion in Abhängigkeit von der Schrittlänge eingestellt.

### Unterscheidung Ebene-Rampe

Der gemessene Absolutwinkel zu Beginn der mittleren Standphase im Gehzyklus, also nach dem Aufsetzen des vollen Fußes auf den Untergrund, ist größer oder kleiner als ein für das Gehen in der Ebene definierter Wertebereich für den Absolutwinkel.

Entsprechend der festgestellten Neigung der Rampe wird der Dorsalanschlag verändert und die Dämpfungseigenschaften bei der Plantarflexion und der Dorsalflexion werden in Abhängigkeit des Absolutwinkels und der prognostizierten Schrittlänge eingestellt.

### Erkennung Rückwärtsgehen

Die Erkennung des Rückwärtsgehens besteht aus einer Erkennung der Rückschwungphase und der Erkennung eines Vorderfußauftritts in dem rückgeführten Zustand.
1. Erkennung einer Rückschwungphase
   Bei einem gemessenen Knöchelmoment nahe Null entspricht das Absolutwinkelsignal einem gegenüber dem Stehen zurückgeführten Fuß (Retroversion) und die Absolutwinkelgeschwindigkeit überschreitet einen definierten Schwellenwert.
2. Erkennung des Vorderfußauftritts im rückgeführten Zustand
   Gemessen wird ein größeres positives Knöchelmoment.
   In Abhängigkeit von den gemessenen Werten wird der Anschlag nach dorsal verstellt und die Dämpfungseigenschaften bei Plantar- und Dorsalflexion werden in Abhängigkeit vom Absolutwinkel bei dem Vorfußauftritt eingestellt.

### Anpassung an unterschiedliche Absatzhöhen

Vorzugsweise wird die Absatzhöhe durch das Auslesen des Absolutwinkelsignals während eines manuell ausgelösten Triggersignals festgestellt. Proportional zum Absolutwinkel wird der Nullpunkt für die Steuerventile 27, 28 eingestellt.

Alternativ hierzu ist es möglich, die Absatzhöhe von einer Rampenneigung bei einem künstlichen Fuß mit einem gelenkig angeschlossenen Vorderfußteil 15 dadurch zu bestimmen, dass der Winkel des Vorderfußteils 15 in Relation zum Hauptfußteil 10 gemessen wird. Hierin liegt eine zusätzliche Option im Rahmen der vorliegenden Erfindung.

### Stehen auf geneigtem Untergrund

Gemessen wird der Absolutwinkel bei einer Umkehr der Bewegungsrichtung von plantar nach dorsal, wenn das Knöchelmoment einen Nulldurchgang erfährt. Dementsprechend wird der dorsale Anschlag für die Steuerung des Hydraulikzylinders 17 mit den Steuerventilen 27, 28 in Abhängigkeit von der Untergrundneigung verstellt.

### Erkennung Treppengehen

Wenn der Absolutwinkelsensor 20 aus zwei Beschleunigungssensoren für senkrecht aufeinanderstehende Beschleunigungskomponenten besteht und die Beschleunigungskomponenten separat ausgebbar sind, kann der vertikal zurückgelegte Weg und der horizontal zurückgelegte Weg des Hauptfußteils 10 festgestellt werden. Die Wegstrecken werden durch zweifache Integration über die entsprechenden Beschleunigungskomponenten ermittelt. In diesen Fällen ist es möglich, eine Unterscheidung von Treppauf- und Treppabgehen vorzunehmen und dementsprechend die Anschläge für die Dämpfungseigenschaften bei Plantar- und Dorsalflexion einzustellen.

In ähnlicher Weise können die Beschleunigungen ausgenutzt werden, um das Gehen bei unterschiedlichen Ganggeschwindigkeiten, einzustellen, indem die Anschläge nach dorsal sowie die Dämpfungseigenschaften bei Plantar- und Dorsalflexion entsprechend geändert werden.

## Patentansprüche

1. Passives orthopädisches Hilfsmittel in Form einer Fußprothese oder Fußorthese mit
(a) einem ersten Teil (10),
(b) einem zweiten Teil (4, 15), wobei das erste Teil (10) drehbar über ein Drehgelenk (5, 14) mit einem zweiten Teil (4, 15) verbunden ist,
(c) einer Sensoranordnung (7, 20, 21) zur Messung von Parametern,
die Rückschlüsse auf momentane Einsatzerfordernisse des Hilfsmittels erlauben,
(d) einer mit der Steueranordnung verbundenen Steuerung zur Bestimmung von Einsatzerfordernissen und Erstellung entsprechender Steuersignale,
(e) einer steuerbaren hydraulischen Dämpferanordnung,
mit der ein auf die Drehbewegung zwischen dem ersten Teil (10) und dem zweiten Teil (4, 15) einwirkender Bewegungswiderstand veränderbar ist, und
(f) einer die Steuersignale der Prozessoranordnung umsetzenden Steurung zur Steuerung der Dämpferanordnung,
wobei
(g) die Dämpferanordnung ein doppelt wirkender Hydraulikzylinder (17) mit zwei durch einen Kolben (18) voneinander getrennten Hydraulikkammern ist und
(h) die Hydraulikkammern über zwei, eine Strömung der Hydraulikflüssigkeit nur in entgegengesetzten Richtungen zulassenden Verbindungsleitungen verbunden sind, deren Strömungswiderstände durch die Steuerung separat mit jeweils einem eigenen Stellmittel einstellbar sind, und
**dadurch gekennzeichnet, dass**
(i) die Prozessoranordnung zur Bestimmung einer aktuellen Nullpunktstellung aus Gemessenen Parametern der wenigstens einen Knöchelmomentsensor (21) und einen Neigungs- oder Absolutwinkelsensor (7, 20) aufweisenden, Sensoranordnung ausgelegt ist.

2. Orthopädietechnisches Hilfsmittel nach Anspruch 1, **dadurch gekennzeichnet, dass** die Erstellung der Steuersignale für die Strömungswiderstände in den beiden Verbindungsleitungen in Bezug auf die Nullpunktstellung erfolgt.

3. Orthopädietechnisches Hilfsmittel nach Anspruch 1 oder 2, **dadurch gekennzeichnet, dass** der Strömungswiderstand in zumindest einer Verbindungsleitung so groß einstellbar ist, dass das Drehgelenk (5, 14) in einer beliebigen Stellung arretierbar ist.

4. Orthopädietechnisches Hilfsmittel nach Anspruch 3, **dadurch gekennzeichnet, dass** die arretierte Stellung der Nullpunktstellung entspricht.

5. Orthopädietechnisches Hilfsmittel nach einem der Ansprüche 1 bis 4, **dadurch gekennzeichnet, dass** die Nullpunktstellung in Abhängigkeit einer Neigung des Untergrunds bestimmbar ist.

6. Orthopädietechnisches Hilfsmittel nach Anspruch 5, **dadurch gekennzeichnet, dass** die Nullpunktstellung in Abhängigkeit von einer aktuellen Absatzhöhe eines mit dem Hilfsmittel verwendeten Schuhs bestimmbar ist.

7. Orthopädietechnisches Hilfsmittel nach einem der Ansprüche 1 bis 6, **dadurch gekennzeichnet, dass** die Teile des Hilfsmittels überwiegend starr ausgebildet sind und dass die Steuerung wenigstens eines überwiegenden Teils des Bewegungsablaufs innerhalb eines Bewegungszyklusses ohne Einfluss von Materialelastizitäten durch die Steuerung der Strömungswiderstände erfolgt.

8. Orthopädietechnisches Hilfsmittel nach einem der Ansprüche 1 bis 7, **dadurch gekennzeichnet, dass** das Drehgelenk ein Knöchelgelen (5) ist.

9. Verfahren zur Steuerung eines orthopädietechnisches Hilfsmittels nach einem der Ansprüche 1 bis 8, **dadurch gekennzeichnet, dass** eine Nullpunktstellung der Drehbewegung bestimmt wird, von der aus bei einem zyklischen Bewegungsablauf der Bewegungswiderstand für mindestens eine Bewegungsrichtung bestimmt wird.

10. Verfahren nach Anspruch 9, **dadurch gekennzeichnet, dass** das Hilfsmittel für ein definiertes Einsatzerfordernis durch Einstellung hoher Bewegungswiderstände in beiden Bewegungsrichtungen in dem Nullpunkt arretiert wird.

11. Verfahren nach Anspruch 8 oder 9, angewendet auf ein orthopädietechnisches Hilfsmittel für eine untere Extremität, bei der ein Gangzyklus als steuerbarer Bewegungszyklus benutzt wird.

## Claims

1. Passive orthopedic aid in the form of a foot prosthesis or foot orthosis, with
(a) a first part (10),
(b) a second part (4, 15), wherein the first part (10) is connected to a second part (4, 15) in a rotatable manner via a swivel joint (5, 14),
(c) a sensor arrangement (7, 20, 21) for measuring parameters,
that provide indications of instantaneous use quirements of the aid,
(d) a control means which is connected to the control arrangement and is used to determine use requirements and to generate corresponding control signals,
(e) a controllable hydraulic damping arrangement,
with which a movement resistance acting on the rotation movement between the first part (10) and the second part (4, 15) can be modified, and
(f) a control means which converts the control signals of the processor arrangement and is used to control the damping arrangement,
wherein
(g) the damping arrangement is a dual-action hydraulic cylinder (17) with two hydraulic chambers separated from each other by a piston (18), and
(h) the hydraulic chambers are connected via two connection lines which permit a flow of the hydraulic fluid only in mutually opposite directions and whose flow resistances can be adjusted by the control separately and in each case via their own adjustment means,
**characterized in that**
(i) the processor arrangement is configured to determine a current neutral point position from the measured parameters of the sensor arrangement having at least one ankle moment sensor (21) and an inclination or absolute angle sensor (7, 20).

2. Orhthopedic aid according to Claim 1, **characterized in that** the control signals for the flow resistances in both connection lines are generated with respect to the neutral position point.

3. Orthopedic aid according to Claim 1 or 2, **characterized in that** the flow resistance in at least one connection line can be made so great that the swivel joint (5, 14) can be locked in any desired position.

4. Orthopedic aid according to Claim 3, **characterized in that** the locked position corresponds to the neutral point position.

5. Orthopedic aid according to one of Claims 1 to 4, **characterized in that** the neutral point position can be determined as a function of an inclination of the ground.

6. Orthopedic aid according to Claim 5, **characterized in that** the neutral point position can be determined as a function of a current height of the heel of a shoe that is used with the aid.

7. Orthopedic aid according to one of Claims 1 to 6, **characterized in that** the parts of the aid are mostly rigid, and **in that** at least most of the movement within a movement cycle is controlled by the control of the flow resistances, without being influenced by material elasticities.

8. Orthopedic aid according to one of Claims 1 to 7, **characterized in that** the swivel joint is an ankle joint (5).

9. Method for controlling an orthopedic aid according to one of Claims 1 to 8, **characterized in that** a neutral point position of the rotation movement is determined from which, in a cyclical movement, the movement resistance for at least one movement direction is determined.

10. Method according to Claim 9, **characterized in that** the aid, for a defined input requirement, is locked in the neutral point by setting high movement resistances in both directions of movement.

11. Method according to Claim 8 or 9, applied to an orthopedic aid for a lower extremity, in which a gait cycle is used as controllable movement cycle.

## Revendications

1. Accessoire orthopédique passif sous la forme d'une prothèse de pied ou d'une orthèse de pied, comprenant
(a) une première partie (10),
(b) une seconde partie (4, 15), la première partie (10) étant reliée en rotation via une articulation rotative (5, 14) avec la seconde partie (4, 15),
(c) un agencement capteur (7, 20, 21) pour la mesure de paramètres, qui permettent de tirer des conclusions sur les exigences d'emploi momentanées de l'accessoire,
(d) une commande, reliée à l'agencement de commande pour la détermination des exigences d'emploi et l'établissement de signaux de commande correspondants,
(e) un agencement amortisseur hydraulique susceptible d'être commandé, au moyen duquel une résistance au mouvement, agissant sur le mouvement de rotation entre la première partie (10) et la seconde partie (4, 15), est susceptible d'être modifiée, et
(f) une commande, mettant en oeuvre les signaux de commande de l'agencement à processeur pour la commande de l'agencement amortisseur,
dans lequel
(g) l'agencement amortisseur est un cylindre hydraulique à double effet (17) avec deux chambres hydrauliques séparées l'une de l'autre par un piston (18), et
(h) les chambres hydrauliques sont reliées via de conduite de liaison permettant un écoulement du liquide hydraulique uniquement dans des directions opposées et dont les résistances à l'écoulement sont susceptibles d'être réglées via la commande séparément avec un organe de positionnement propre respectif, et
**caractérisé en ce que**
(i) l'agencement à processeur est conçu pour déterminer une position de point zéro actuelle à partir de paramètres mesurés de l'agencement capteur, qui comprend au moins un capteur de couple à la cheville (21) et un capteur d'angle d'inclinaison ou capteur d'angle absolu (7, 20).

2. Accessoire orthopédique selon la revendication 1, **caractérisé en ce que** l'établissement des signaux de commande pour les résistances à l'écoulement a lieu dans les deux conduites de liaison par référence à la position de point zéro.

3. Accessoire orthopédique selon la revendication 1 ou 2, **caractérisé en ce que** la résistance à l'écoulement dans au moins une conduite de liaison est susceptible d'être réglée si élevée que l'articulation rotative (5, 14) est susceptible d'être arrêtée dans une position quelconque.

4. Accessoire orthopédique selon la revendication 3, **caractérisé en ce que** la position arrêtée correspond à la position au point zéro.

5. Accessoire orthopédique selon l'une des revendications 1 à 4, **caractérisé en ce que** la position de point zéro est susceptible d'être déterminée en fonction d'une inclinaison du sous-sol.

6. Accessoire orthopédique selon la revendication 5, **caractérisé en ce que** la position de point zéro est susceptible d'être déterminée en fonction d'une hauteur de talon actuelle d'une chaussure utilisée avec l'accessoire.

7. Accessoire orthopédique selon l'une des revendications 1 à 6, **caractérisé en ce que** les parties de l'accessoire sont réalisées en majeure partie rigides, et **en ce que** la commande d'au moins une majeure partie du déroulement des mouvements à l'intérieur d'un cycle de mouvement a lieu sans influence des élasticités des matériaux grâce à la commande des résistances à l'écoulement.

8. Accessoire orthopédique selon l'une des revendications 1 à 7, **caractérisé en ce que** l'articulation rotative est une articulation de la cheville (5).

9. Procédé pour la commande d'un accessoire orthopédique selon l'une des revendications 1 à 8, **caractérisé en ce que** l'on détermine une position de point zéro du mouvement rotatif, à partir de laquelle on détermine, pour un déroulement cyclique du mouvement, la résistance au mouvement pour au moins une direction de mouvement.

10. Procédé selon la revendication 9, **caractérisé en ce que** l'accessoire est arrêté au point zéro, pour une exigence d'emploi définie, par réglage de résistances aux mouvements élevées dans les deux directions de mouvement.

11. Procédé selon la revendication 8 ou 9, appliqué à un accessoire orthopédique pour une extrémité inférieure, dans lequel le cycle de marche est utilisé à titre de cycle de mouvement à commander.
